# EUROPÄISCHE PATENTANMELDUNG

(11) **EP 0 941 990 A2**
(43) Veröffentlichungstag der Anmeldung: **15.09.1999**
(21) Anmeldenummer: 99103880.3
(22) Anmeldetag: 01.03.1999
(51) Int. Cl.: C07D 277/42, C07D 293/06, C07C 255/34

(54) **Verfahren zur Herstellung von Azamethinen sowie Azamethine selbst**

(30) Priorität: 09.03.1998 DE 19810063
(71) Anmelder: SIEMENS AKTIENGESELLSCHAFT, 80333 München (DE)
(72) Erfinder: Kanitz, Andreas, Dr., 91333 Höchstadt (DE); Hartmann, Horst, Prof., Dr., 06217 Merseburg (DE); Fricke, Christian, Dr., 14050 Berlin (DE)

(57) **Zusammenfassung**

Die Erfindung betrifft ein Verfahren zur Herstellung von Azamethinen der allgemeinen Formel 1 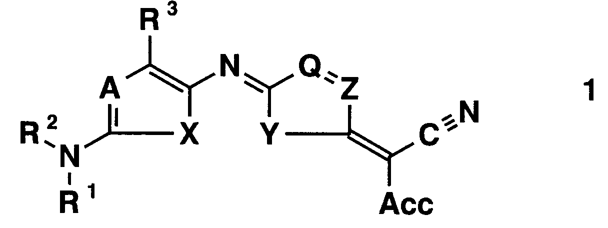 durch Kondensation von Verbindungen der allgemeinen Formel 2 mit methylenaktiven Verbindungen der allgemeinen Formel 3A/3B gemäß folgendem Reaktionsschema 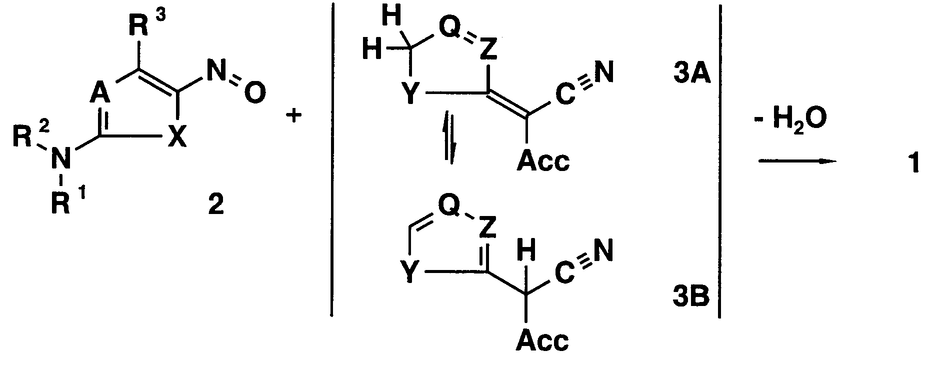 in einem Lösungsmittel bei einer Temperatur zwischen 60 und 120°C und anschließender Isolierung der Produkte. Die Erfindung betrifft auch neue Azamethine, insbesondere solche mit heterocyclischen Strukturelementen. Diese Azamethine eignen sich aufgrund ihrer Eigenschaften zur Verwendung als nichtlinear-optische chromophore Bausteine.

## Beschreibung

Die vorliegende Erfindung betrifft ein Verfahren zur Herstellung von bisher größtenteils unbekannten Azamethinen der allgemeinen Formel 1 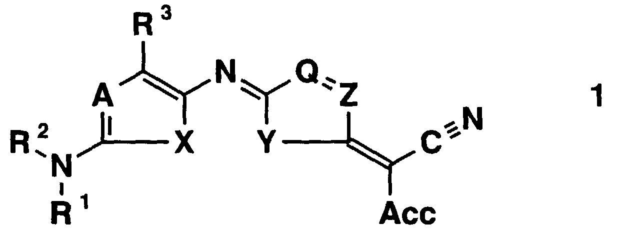 sowie die neuen Azamethine selbst.

Vom Verbindungstyp 1 sind bisher lediglich solche Verbindungen bekannt, in denen X und Y jeweils eine Ringdoppelbindung bedeuten, d.h. eine CH=CH-Gruppierung, A eine CH-Gruppe ist und Q und Z zusammen einen 1,2-anellierten Benzolring bilden. Diese aus dem Stand der Technik bekannten Azamethine werden durch Kupplung von N,N-disubstituierten para-Phenylendiaminen mit 1-Dicyanomethyl-naphthalin in Gegenwart eines geeigneten Oxidationsmittels, wie Natriumhypochlorit, Kaliumdichromat oder Kaliumferrocyanid, hergestellt (Y. Kubo, F. Mori, K. Komatsu, K. Yoshida in J. Chem. Soc., Perkin Trans. 1, 1988, Seiten 2439 bis 2442; Y. Kubo, M. Kuwana, K. Yoshida, Y. Tomotake, T. Matsuzaki, S. Maeda in J. Chem. Soc., Chem. Commun., 1989, Seiten 35 bis 37). Solche Farbstoffe wurden zur Herstellung von nichtlinear-optischen Materialien (S. Aramaki, Y. Okamoto, T. Murayama, Y. Kubo in SPIE, Vol. 2285 (1994), Seiten 58 bis 66) und als Entwicklungsfarbstoffe für den nahen Infrarot-Spektralbereich (Y. Kubo in J. Chem. Soc., Perkin Trans. 1, 1994, Seiten 1787 bis 1789) vorgeschlagen.

Das aus dem Stand der Technik bekannte Herstellungsverfahren für den genannten Verbindungstyp weist jedoch erhebliche Nachteile auf, die vor allem darin bestehen, daß einerseits para-Phenylendiamin-Derivate als Kupplungskomponenten und andererseits Oxidationsmittel als Reaktionskomponenten eingesetzt werden müssen. Daher ist die Auswahl an einsetzbaren Edukten eingeschränkt, da viele der Verbindungen unter den oxidativen Kupplungsbedingungen anderweitige Oxidations- oder Zersetzungsreaktionen eingehen. Auch ist eine Ausdehnung des bekannten Verfahrens auf Azamethine mit vorzugsweise heterocyclischen Strukturelementen nicht möglich, da die hierfür erforderlichen heterocyclischen Diamin-Derivate in der Regel nicht oder nur schwer zugänglich sind oder nicht in kupplungsfähige heteroanaloge Chinondiiminiumsalze überführbar sind.

Vorrangige Aufgabe der vorliegenden Erfindung ist es daher, ein Verfahren an die Hand zu geben, mit dem auf einfache und kostengünstige Weise Azamethine der allgemeinen Formel 1 aus leicht zugänglichen Ausgangsstoffen und in hoher Reinheit hergestellt werden können. Auch ist es Aufgabe der vorliegenden Erfindung, neue Azamethine der allgemeinen Formel 1 mit vorzugsweise heterocyclischen Strukturelementen zur Verfügung zu stellen, welche als nichtlinear-optische chromophore Bausteine Verwendung finden können.

Überraschenderweise konnten diese Aufgaben dadurch gelöst werden, daß man gemäß der folgenden Reaktionsgleichung 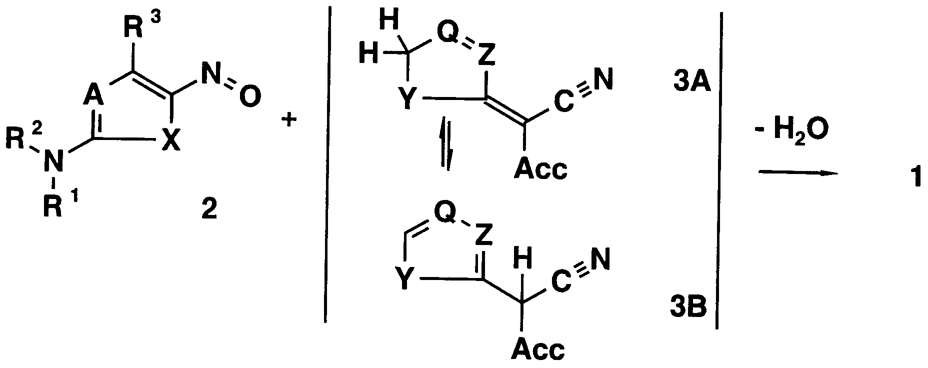 eine Verbindung der allgemeinen Formel 2 mit einer Verbindung der allgemeinen Formel 3A/3B in einem Lösungsmittel bei einer Temperatur zwischen 60 und 120°C kondensiert und das Kondensationsprodukt isoliert,
wobei in den Formeln 1 und 2
R¹ und R² - gleich oder verschieden voneinander - jeweils ein Wasserstoffatom, ein gerader oder verzweigter C₁- bis C₂₀-Alkylrest, ein Phenyl-, Naphthyl-, Thienyl-, Thiazolyl- oder Pyridylrest sind oder zusammen einen fünf- oder sechsgliedrigen carbocyclischen oder heterocyclischen Ring ausbilden können;
R³ ein Wasserstoffatom, eine Hydroxyl- oder O-Acylgruppe, ein gerader oder verzweigter C₁- bis C₂₀-Alkylrest oder ein - gegebenenfalls in para-Position mit einer Halogen-, Hydroxyl- oder O-Acylgruppe substituierter - Phenylrest ist;
X für S, Se, O oder NR⁴, wobei R⁴ ein Wasserstoffatom oder ein gerader oder verzweigter C₁- bis C₂₀-Alkylrest ist, oder für eine Ringdoppelbindung oder einen 1,2-anellierten Benzolring steht;
A für eine CH- oder CR⁵-Gruppe, wobei R⁵ ein gerader oder verzweigter C₁- bis C₂₀-Alkylrest, ein Phenyl- oder ein Naphthylrest ist, oder für N steht;
und in den Formeln 1 und 3A/3B
Y für S, Se, O oder NR⁶, wobei R⁶ ein Wasserstoffatom oder ein gerader oder verzweigter C₁- bis C₂₀-Alkylrest ist, oder für eine Ringdoppelbindung oder einen 1,2-anellierten Benzolring steht;
Q und Z - gleich oder verschieden voneinander - jeweils für eine CH- oder CR⁷-Gruppe, wobei R⁷ ein C₁- bis C₂₀-Alkylrest, ein gegebenenfalls substituierter Phenyl- oder ein Naphthylrest ist, oder für N stehen oder zusammen für eine Ringdoppelbindung oder einen 1,2-anellierten Benzolring stehen, mit der Maßgabe, daß Y nicht gleichzeitig ein 1,2-anellierter Benzolring ist, und
Acc eine Nitril-, C₁- bis C₂₀-Alkoxycarbonyl-, Formyl-, Acyl-, Arylsulfonyl- oder Nitrogruppe ist.

Bei einer bevorzugten und geeigneten Ausführungsform des erfindungsgemäßen Verfahrens setzt man als Lösungsmittel für die Kondensation Anhydride niederer Carbonsäuren oder dipolare aprotische Lösungsmittel ein oder Säurehalogenide in einem inerten Lösungsmittel. Bei einer besonders bevorzugten Ausführungsform des erfindungsgemäßen Verfahrens wählt man das Lösungsmittel für die Kondensation aus Acetanhydrid, Dimethylformamid, Dimethylacetamid oder N-Methylpyrrolidon oder aus Phosphoroxychlorid oder Thionylchlorid in Dichlorethan oder Perchlorethylen aus.

Bei einer weiteren geeigneten Ausführungsform des erfindungsgemäßen Verfahrens kann man das bei der Herstellung einer Verbindung der allgemeinen Formel 2 anfallende mineralsaure Salz der Verbindung 2 (2·HAn) zur Kondensation einsetzen, wobei man dann während der Kondensation wenigstens eine äquivalente Menge einer Base zur Bindung der Säure zusetzt. Diese Base ist vorzugsweise Triethylamin oder Natriumacetat.

In diesem Zusammenhang muß erwähnt werden, daß unter mineralsauren Salzen (2·HAn) solche der vorgenannten Verbindungen 2 zu verstehen sind, in denen das Anion An⁻ das Salz einer Mineralsäure, wie Chlorid, Bromid oder Iodid sowie Perchlorat, Tetrafluoroborat oder Tetraphenylborat, ist. Bevorzugte Anionen sind die Chloride, Perchlorate und Tetrafluoroborate, da sie während des Herstellungsverfahrens am besten auskri

stallisieren. Bei einer weiteren bevorzugten Ausführungsform des Verfahrens nach der vorliegenden Erfindung setzt man Verbindungen der Struktur 2 bzw. 2·HAn ein, in welchen R¹ und R² gerade oder verzweigte C₁- bis C₂₀-Alkylreste sind, welche - gleich oder verschieden voneinander - durch 1 bis 5 Sauerstoffatome in Etherfunktion unterbrochen sind.

Bei einer weiteren bevorzugten Ausführungsform des erfindungsgemäßen Verfahrens setzt man Verbindungen der Struktur 2 bzw. 2·HAn ein, in welchen ein aus R¹ und R² gebildeter carbocyclischer oder heterocyclischer 5- bzw. 6-Ring mit Hydroxy- und/oder C₁- bis C₂₀-Alkoxyresten substituiert ist.

Bei einer weiteren bevorzugten Ausführungsform des erfindungsgemäßen Verfahrens setzt man Verbindungen der Struktur 2 bzw. 2·HAn ein, in welchen R¹ und R² zusammen einen Morpholin-, Piperidin-, Pyrrolidin- oder Piperazinring bilden oder bilden können.

Bei einer weiteren bevorzugten Ausführungsform des Verfahrens nach der vorliegenden Erfindung setzt man Verbindungen der Struktur 2 bzw. 2·HAn ein, worin R¹ und R² zur Ausbildung eines Morpholin-4-yl-, Piperazin-1-yl- oder 4-(C₁- bis C₄-Alkyl)-piperazin-1-yl-Restes fähig sind. Ganz besonders bevorzugte Verbindungen der allgemeinen Struktur 2 bzw. 2·HAn sind solche, in denen R¹ und R² zur Ausbildung eines 4-Methylpiperazin-1-yl- oder 4-(2-Hydroxyethyl)-piperazin-1-yl-Restes fähig sind.

Des weiteren werden im Rahmen der Erfindung bevorzugt solche Verbindungen der Struktur 2 bzw. 2·HAn eingesetzt, in welchen der Rest R³ ein Wasserstoffatom oder einen substituierten Phenylrest darstellt.

Besonders bevorzugt werden - im Hinblick auf das Endprodukt - Verbindungen der Struktur 2 bzw. 2·HAn eingesetzt, in welchen X für S, Se oder N steht.

Bevorzugte Verbindungen der Struktur 3A/3B, welche bei dem erfindungsgemäßen Verfahren eingesetzt werden, sind - ebenfalls im Hinblick auf ein heterocyclisches Endprodukt - solche, in denen Y für S oder Se steht.

Bei den obengenannten Verbindungen der Struktur 3A/3B, welche bei dem erfindungsgemäßen Verfahren eingesetzt werden, bilden Q und Z bevorzugt zusammen eine Ringdoppelbindung oder einen 1,2-anellierten Benzolring oder Z steht für N und Q für eine CR⁷-Gruppe, wobei R⁷ ein Phenylrest ist, der mit einer NH₂-, NO₂-, OH-, O-Acyl oder COOH-Gruppe substituiert sein kann.

Wiederum im Hinblick auf das Endprodukt werden bei dem Verfahren nach der vorliegenden Erfindung insbesondere solche Verbindungen der Struktur 3A/3B eingesetzt, in welchen Acc eine Nitrilgruppe ist.

Mit dem Verfahren nach der vorliegenden Erfindung lassen sich Azamethine der allgemeinen Formel 1 überraschend in einfacher und kostengünstiger Weise aus leicht zugänglichen Ausgangsprodukten herstellen. Es treten keine unerwünschten Nebenreaktionen ein, welche die Ausbeute an dem gewünschten Endprodukt beeinträchtigen und/oder vermindern. Es können insbesondere oxidationsempfindliche Edukte mit heterocyclischen Strukturelementen oder heterocyclischen Resten eingesetzt werden. Es sind insbesondere solche Verbindungen der allgemeinen Formel 1 erhältlich, welche aufgrund ihres Substitutionsmusters als nichtlinear-optische chromophore Bausteine in NLO-aktiven Bauelementen eingesetzt werden können. Die Grundstruktur der Azamethine sowie ihr Substitutionsmuster sind dabei so ausgewählt, daß die Azamethine aufgrund ihrer chromophoren Eigenschaften (Push-Pull-System) sowie ihrer sonstigen chemischen bzw. physikalischen Eigenschaften in Polymere eingebettet bzw. daran kovalent gebunden werden können, welche sich für den Aufbau von NLO-aktiven Bauelementen eignen.

Gegenstand der vorliegenden Erfindung sind auch Azamethine der folgenden allgemeinen Formel 1 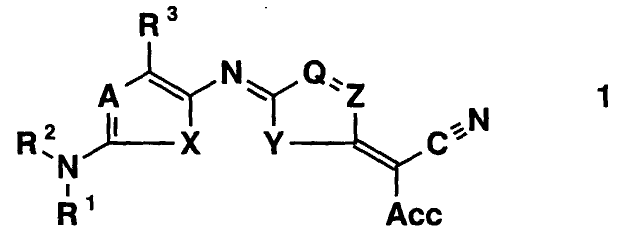 worin
R¹ und R² - gleich oder verschieden voneinander - jeweils ein Wasserstoffatom, ein gerader oder verzweigter C₁- bis C₂₀-Alkylrest, ein Phenyl-, Naphthyl-, Thienyl-, Thiazolyl- oder Pyridylrest sind oder zusammen einen fünf- oder sechsgliedrigen carbocyclischen oder heterocyclischen Ring ausbilden können;
R³ ein Wasserstoffatom, eine Hydroxyl- oder O-Acylgruppe, ein gerader oder verzweigter C₁- bis C₂₀-Alkylrest oder ein - gegebenenfalls in para-Position mit einer Halogen-, Hydroxyl- oder O-Acylgruppe substituierter - Phenylrest ist;
X für S, Se, O oder NR⁴, wobei R⁴ ein Wasserstoffatom oder ein gerader oder verzweigter C₁- bis C₂₀-Alkylrest ist, oder für einen 1,2-anellierten Benzolring steht;
A für eine CH- oder CR⁵-Gruppe, wobei R⁵ ein gerader oder verzweigter C₁- bis C₂₀-Alkylrest, ein Phenyl- oder ein Naphthylrest ist, oder für N steht;
Y für S, Se, O oder NR⁶, wobei R⁶ ein Wasserstoffatom oder ein gerader oder verzweigter C₁- bis C₂₀-Alkylrest ist, oder für einen 1,2-anellierten Benzolring steht;
Q und Z - gleich oder verschieden voneinander - jeweils für eine CH- oder CR⁷-Gruppe, wobei R⁷ ein C₁- bis C₂₀-Alkylrest, ein gegebenenfalls substituierter Phenyl- oder ein Naphthylrest ist, oder für N stehen oder zusammen für eine Ringdoppelbindung stehen, und
Acc eine Nitril-, C₁- bis C₂₀-Alkoxycarbonyl-, Formyl-, Acyl-, Phenylsulfonyl-, Naphthylsulfonyl- oder Nitrogruppe ist.

Hierbei sind solche Verbindungen bevorzugt, in denen R¹ und R² gerade oder verzweigte C₁- bis C₂₀-Alkylreste sind, welche - gleich oder verschieden voneinander - durch 1 bis 5 Sauerstoffatome in Etherfunktion unterbrochen sind.

Vorzugsweise sind die aus R¹ und R² geformten bzw. im Laufe des Herstellungsverfahrens gebildeten carbocyclischen oder heterocyclischen Ringe zusätzlich mit Hydroxy- und/oder C₁- bis C₂₀-Alkoxyresten substituiert.

R¹ und R² bilden zusammen bevorzugt einen Morpholin-, Piperidin-, Pyrrolidin- oder Piperazinring oder sind in der Lage, einen derartigen Ring zu bilden. Bevorzugte Ringsysteme sind hierbei ein Morpholin-4-yl-, Piperazin-1-yl- oder 4-(C₁- bis C₄-Alkyl)-piperazin-1-yl-Rest und insbesondere ein 4-Methylpiperazin-1-yl- oder 4-(2-Hydroxyethyl)-piperazin-1-yl-Rest.

Bevorzugte Verbindungen sind des weiteren solche, in denen R³ ein Wasserstoffatom oder ein substituierter Phenylrest ist, X für S, Se oder N steht, Y für S oder Se steht und/oder Q und Z zusammen eine Ringdoppelbindung bilden oder Z für N und Q für eine CR⁷-Gruppe steht, wobei R⁷ ein gegebenenfalls substituierter Phenylrest ist.

Ganz besonders bevorzugte Azamethine sind ferner diejenigen, welche in den nachfolgend angegebenen Beispielen 14 bis 31 beschrieben sind.

Bei der Herstellung der Azamethine werden die beschriebenen Edukte vorzugsweise im äquimolaren Verhältnis miteinander umgesetzt, und die gebildeten Produkte werden vorzugsweise durch Verdünnen der Reaktionslösung mit einem weniger polaren Lösungsmittel, wie Diethylether oder ein C₁- bis C₅-Alkohol, ausgefällt und durch Filtrieren, Umkristallisieren oder bekannte und geeignete Chromatographieverfahren isoliert.

Im weiteren soll die Erfindung durch die nachfolgenden Beispiele näher erläutert werden, wobei zunächst die Herstellung der für die Synthese der Azamethine 1 erforderlichen Nitrosoverbindungen 2 oder deren mineralsauren Salze 2·HAn (Beispiele 1 bis 7), danach die Synthese der methylenaktiven Cyanverbindungen des Formeltyps 3A/3B (Beispiele 8 bis 13) und im Anschluß daran die Synthese der unter anderem beanspruchten Azamethin-Derivate 1 (Beispiele 14 bis 31) beispielhaft beschrieben wird.

In den Beispielen werden folgende Abkürzungen verwendet:
- Fp. = Fusionspunkt (Schmelzpunkt);
- Ausb. = Ausbeute;
- Zers. = Zersetzung.

### Beispiel 1

### 5-N,N-Diethylamino-2-nitroso-phenol-hydrochlorid

0,1 mol 3-Diethylamino-phenol werden in 50 ml mit gasförmigem Chlorwasserstoff gesättigtem n-Propanol gelöst und bei 0°C unter Rühren tropfenweise mit 0,1 mol Isoamylnitrit versetzt. Nach weiterem einstündigem Rühren wird die Reaktionslösung mit Diethylether versetzt und das gebildete Produkt durch Absaugen isoliert.
Ausb. 71 %; Fp. 175 - 177°C.

### Beispiel 2

### 5-N,N-Di(n-butylamino)-2-nitroso-phenol-hydrochlorid

Analog Beispiel 1 wird aus 0,1 mol 3-(N,N-Dibutylamino)-phenol und 0,1 mol Isoamylnitrit die Titelverbindung erhalten.
Ausb. 53 %; Fp. 160°C (Zers.).

### Beispiel 3

### 5-[N-Butyl-N-(2-hydroxyethyl)-amino]-2-nitroso-phenol-hydrochlorid

Analog Beispiel 1 wird aus 0,1 mol 3-[N-Butyl-N-(2-hydroxyethyl)-amino]-phenol und 0,1 mol Isoamylnitrit die Titelverbindung erhalten.
Ausb. 76 %; Fp. 127°C (Zers.).

### Beispiel 4

### 5-[N-Heptyl-N-(2-hydroxyethyl)-amino]-2-nitroso-phenol-hydrochlorid

Analog Beispiel 1 wird aus 0,1 mol 3-[N-Heptyl-N-(2-hydroxyethyl)-amino]-phenol und 0,1 mol Isoamylnitrit die Titelverbindung erhalten.
Ausb. 62 %; Fp. 129°C (Zers.).

### Beispiel 5

### 4-Nitroso-N,N-dimethyl-1-naphthylamin-hydroperchlorat

Analog Beispiel 1 wird aus 0,1 mol N,N-Dimethyl-1-naphthylamin und 0,1 mol Natriumnitrit - unter Verwendung von halbkonzentrierter Salzsäure als Reaktionsmedium - die entsprechende Nitrosoverbindung hergestellt, die anschließend durch Zugabe von 70%iger Perchlorsäure als Hydroperchlorat ausgefällt wird.
Ausb. 92%; Fp. 169 - 171°C.

### Beispiel 6

### 4-Nitroso-N-ethyl-1-naphthylamin-hydroperchlorat

Analog Beispiel 5 wird aus 0,1 mol N-Ethyl-1-naphthylamin und 0,1 mol Natriumnitrit die Titelverbindung unter Verwendung von Nitrosylschwefelsäure (hergestellt durch Eintragen von 0,1 mol Natriumnitrit in 150 ml konz. Schwefelsäure) als Reaktionsmedium und Fällen des gebildeten Nitrosoprodukts - nach dessen Einrühren in 500 g Eiswasser - durch Zugabe von Perchlorsäure hergestellt.
Ausb. 80 %; Fp. 183 - 165°C.

### Beispiel 7

### 2-Morpholino-4-phenyl-5-nitroso-thiazol

Analog Beispiel 1 wird aus 0,1 mol 2-Morpholino-4-phenylthiazol und 0,1 mol Isoamylnitrit die Titelverbindung erhalten.
Ausb. 73%; Fp. 174 - 175°C.

### Beispiel 8

### 1-Naphthyl-malonsäuredinitril

Zu einer Lösung von 0,65 mol Lithium-di-isopropylamid in Benzol werden unter Rühren und Kühlen 0,3 mol 1-Naphthylacetonitril und anschließend tropfenweise 0,6 mol 2-Chlorbenzylrhodanid zugefügt. Nach einer Stunde wird die Reaktionsmischung in 1 l 0,3 molare NaOH eingerührt und nach einigem Stehen in die beiden sich bildenden Phasen getrennt. Die wäßrige Lösung wird durch Zugabe von Salzsäure neutralisiert und über Nacht stehen gelassen. Dann wird das ausgefallene Produkt abgesaugt, getrocknet und durch Umkristallisation aus Ethanol gereinigt.
Ausb. 32 %; Fp. 165°C.

### Beispiel 9

### 2-Thienyl-malonsäuredinitril

### Variante a:

Analog Beispiel 8 wird aus 0,1 mol 2-Thienyl-acetonitril, 0,3 mol Lithium-di-isopropylamid und 0,2 mol 2-Chlorbenzylrhodanid die Titelverbindung in Form eines farblosen Öls erhalten.
Ausb. 24 %.

### Variante b:

0,13 mol Malonsäuredinitril werden in 100 ml wasserfreiem Tetrahydrofuran unter Rühren und Kühlen portionsweise mit 0,15 mol Natriumhydrid versetzt. Anschließend werden 0,0015 mol Palladiumchlorid-Triphenylphosphin-Komplex zugegeben und 25 g 2-Iod-thiophen tropfenweise zugesetzt, und die Mischung wird dann 3 h unter Rückfluß erhitzt. Nach dem Erkalten wird mit Salzsäure neutralisiert und mit Diethylether extrahiert. Nach dem Abtrennen und Trocknen der etherischen Lösung wird der Ether abdestilliert; das verbleibende ölige Reaktionsprodukt wird für die weiteren Umsetzungen eingesetzt.
Ausb. 24 %.

### Beispiel 10

### 2-Dicyanomethylen-4-phenyl-2,5-dihydro-thiazol

Zu einer Lösung von 0,2 mol ω-Rhodano-acetophenon in 200 ml Ethanol werden 0,2 mol Malonsäuredinitril gegeben, und nach Zugabe von 20 ml Triethylamin wird 30 min unter Rückfluß erhitzt. Nach dem Abkühlen wird mit Eisessig angesäuert und das dabei auskristallisierende Produkt durch Absaugen isoliert.
Ausb. 85 %; Fp. 250 - 252°C.

### Beispiel 11

### 2-Dicyanomethylen-4-(4-nitro-phenyl)-2,5-dihydro-thiazol

Analog Beispiel 10 wird - ausgehend von ω-Rhodano-4-nitroacetophenon - die Titelverbindung erhalten.
Ausb. 92 %; Fp. 265°C (Zers.).

### Beispiel 12

### 2-Dicyanomethylen-4-(4-acetoxy-phenyl)-2,5-dihydro-thiazol

Analog Beispiel 10 wird - ausgehend von ω-Rhodano-4-acetoxyacetophenon - die Titelverbindung erhalten.
Ausb. 82%; Fp. 175°C (Zers.).

### Beispiel 13

### 2-Dicyanomethylen-4-(4-chlor-phenyl)-2,5-dihydro-selenazol

Analog Beispiel 10 wird - ausgehend von ω-Selenocyanato-4-chlor-acetophenon - die Titelverbindung erhalten.
Ausb. 35 %; Fp. 255°C.

### Beispiel 14

### 2-Dicyanomethylen-4-phenyl-5-(4-dimethylamino-phenylimino)-2,5-dihydro-thiazol

0,01 mol 4-Nitroso-N,N-dimethylanilin werden mit 0,01 mol 2-Dicyanomethylen-4-phenyl-2,5-dihydro-thiazol (siehe Beispiel 10) in 50 ml Acetanhydrid einige Minuten auf dem Wasserbad erwärmt. Der nach dem Erkalten ausfallende Feststoff (mit der nachfolgend wiedergegebenen Struktur) wird abgesaugt und aus Eisessig umkristallisiert.
Ausb. 75%; Fp. 264 - 265°C; λₘₐₓ (in CH₂Cl₂): 660 nm, (lg ε: 4,58).

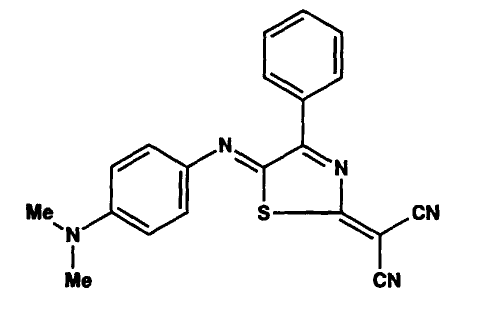

### Beispiele 15 bis 31

Analog Beispiel 14 werden folgende Verbindungen erhalten:

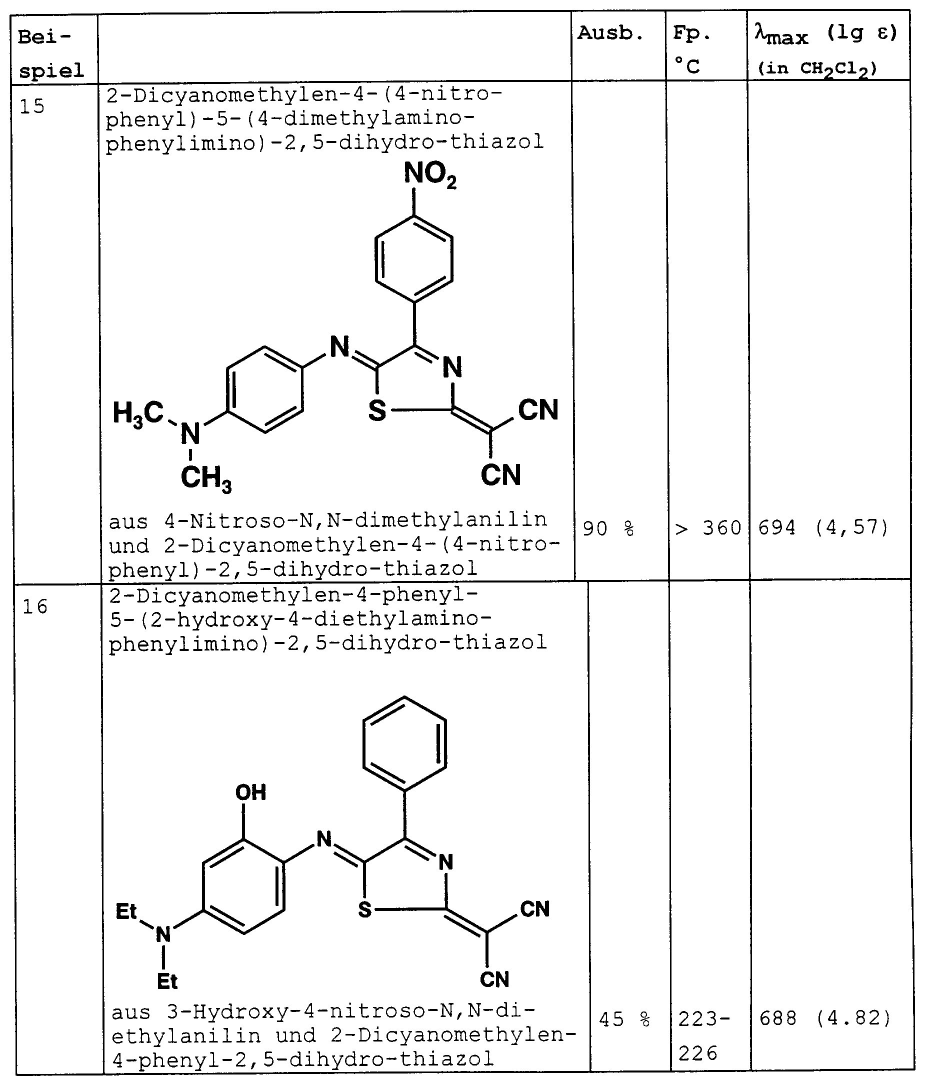

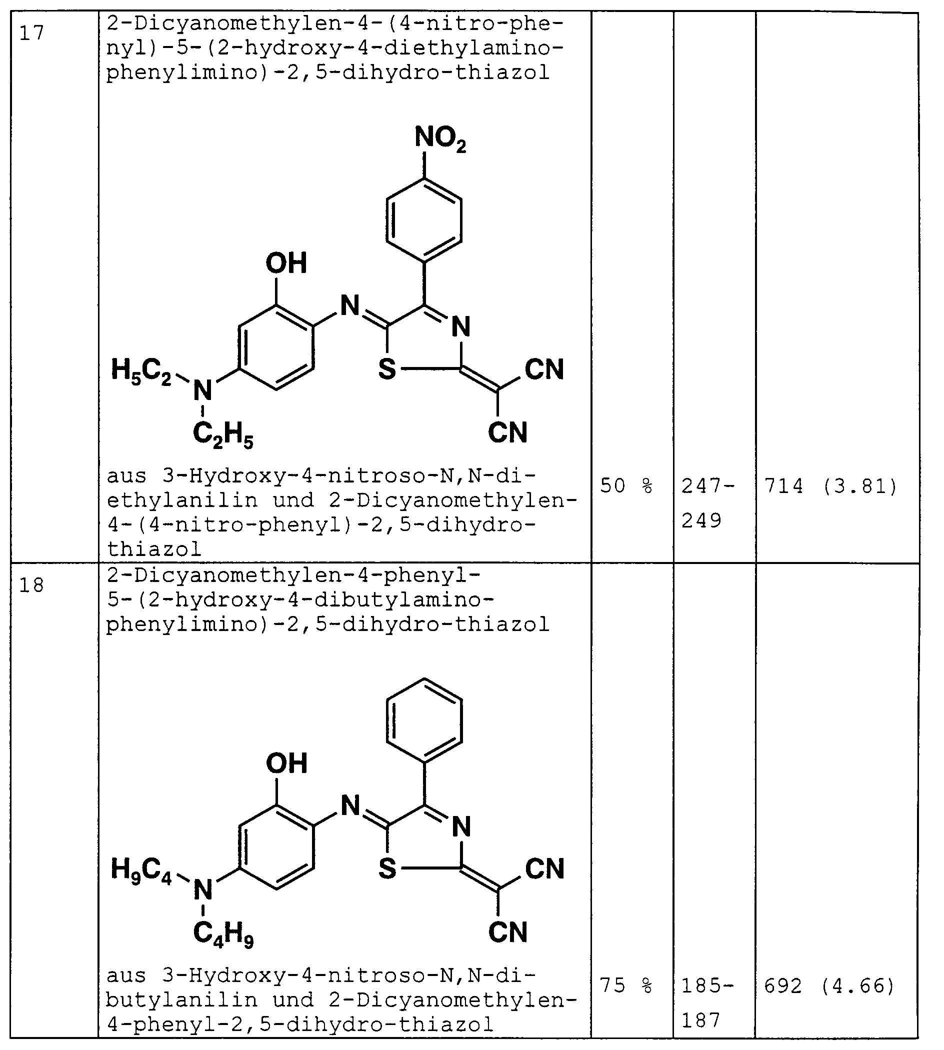

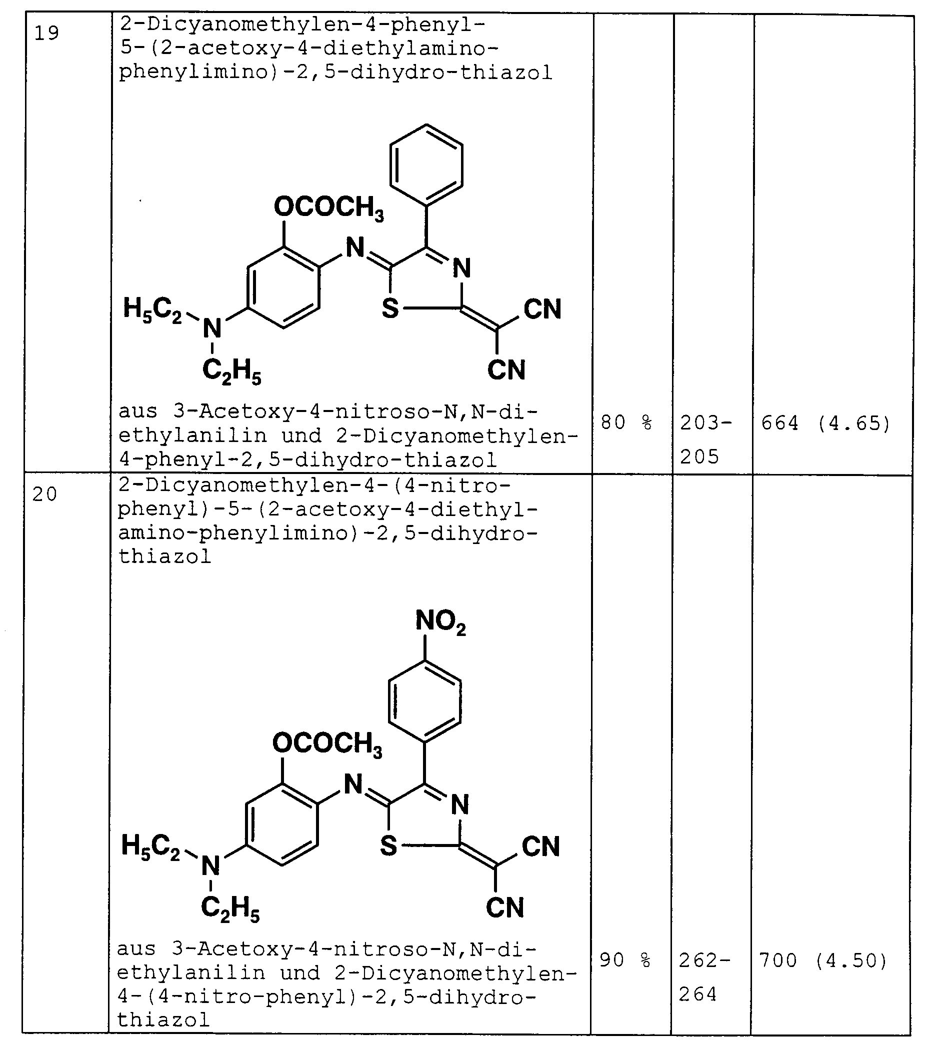

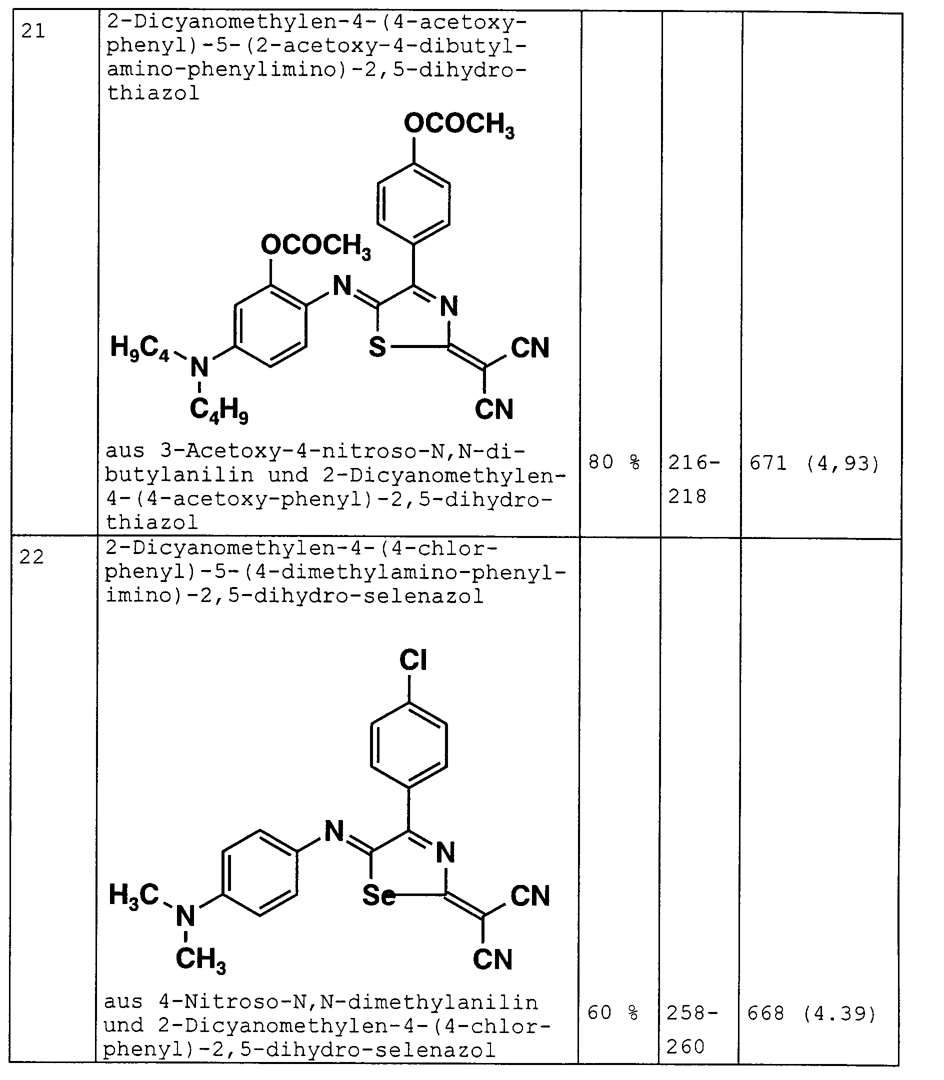

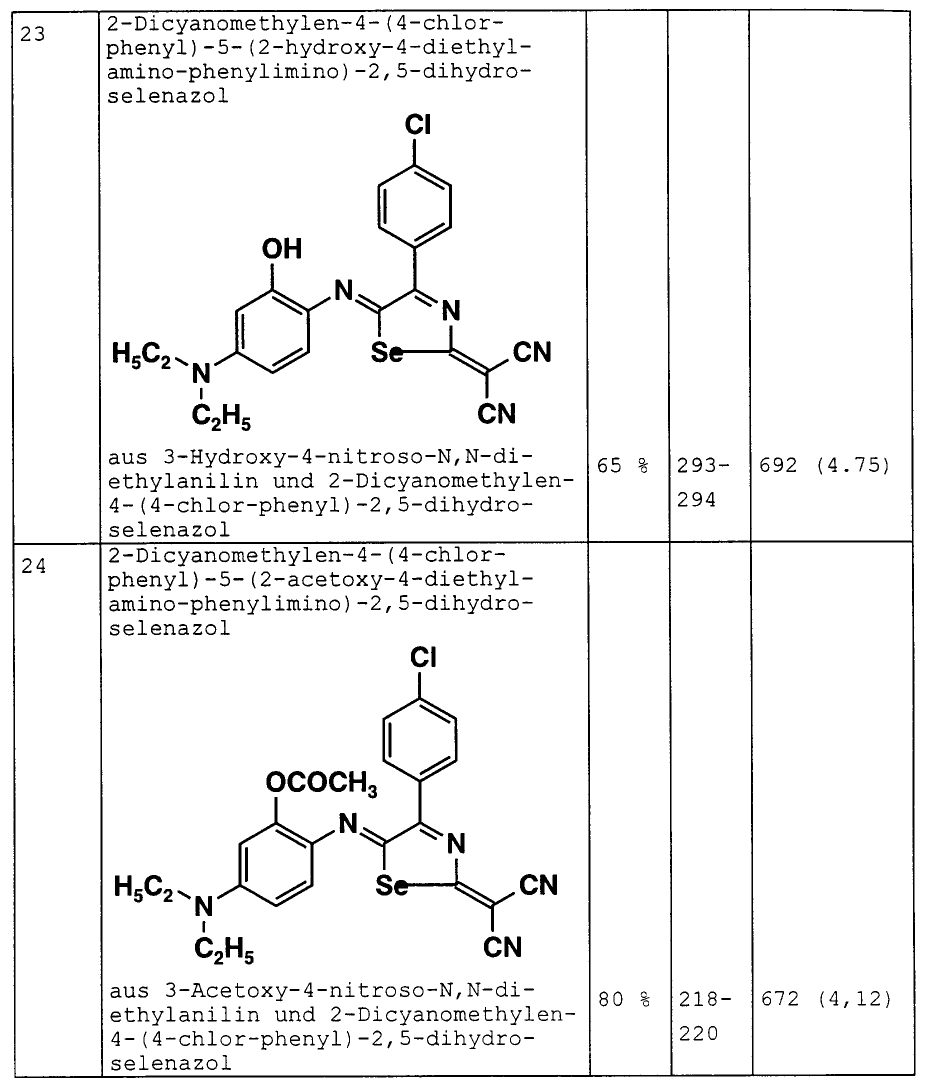

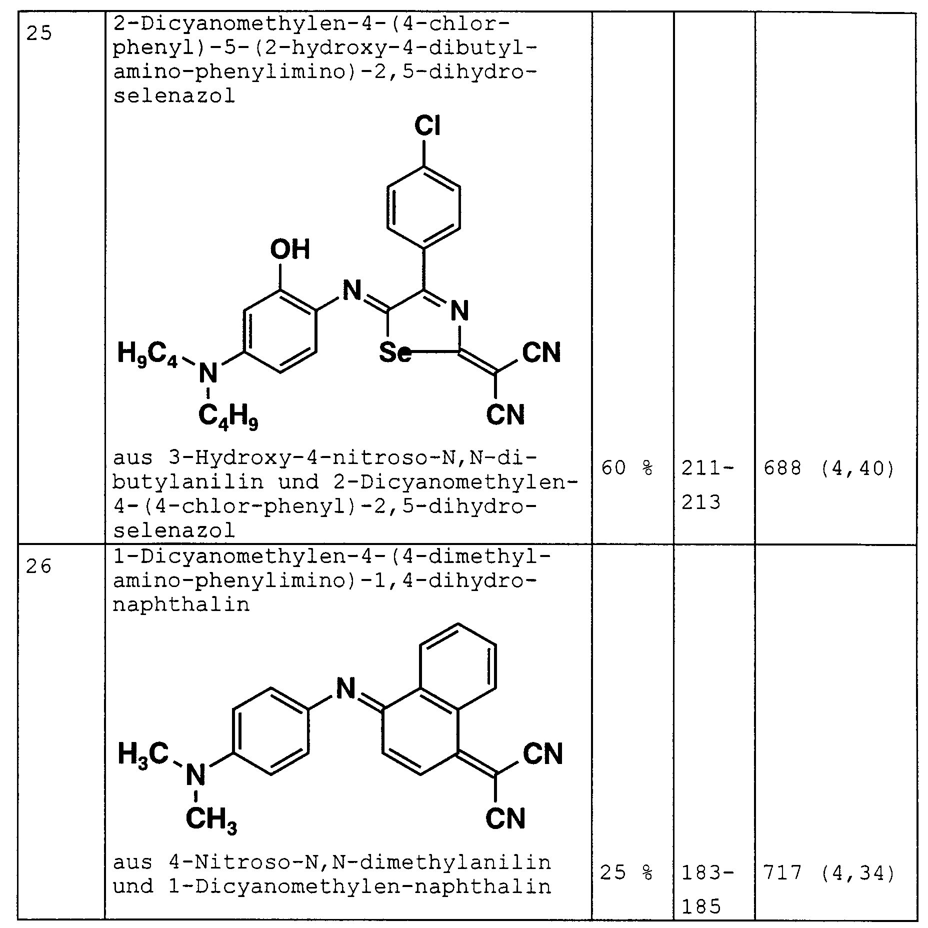

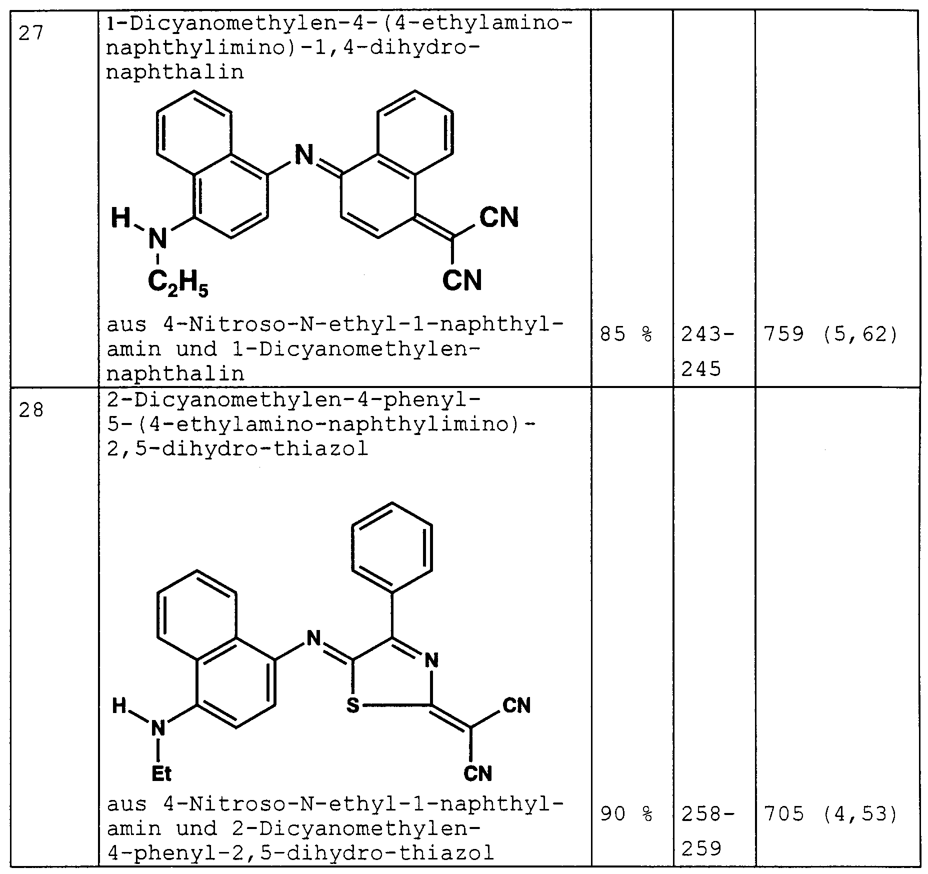

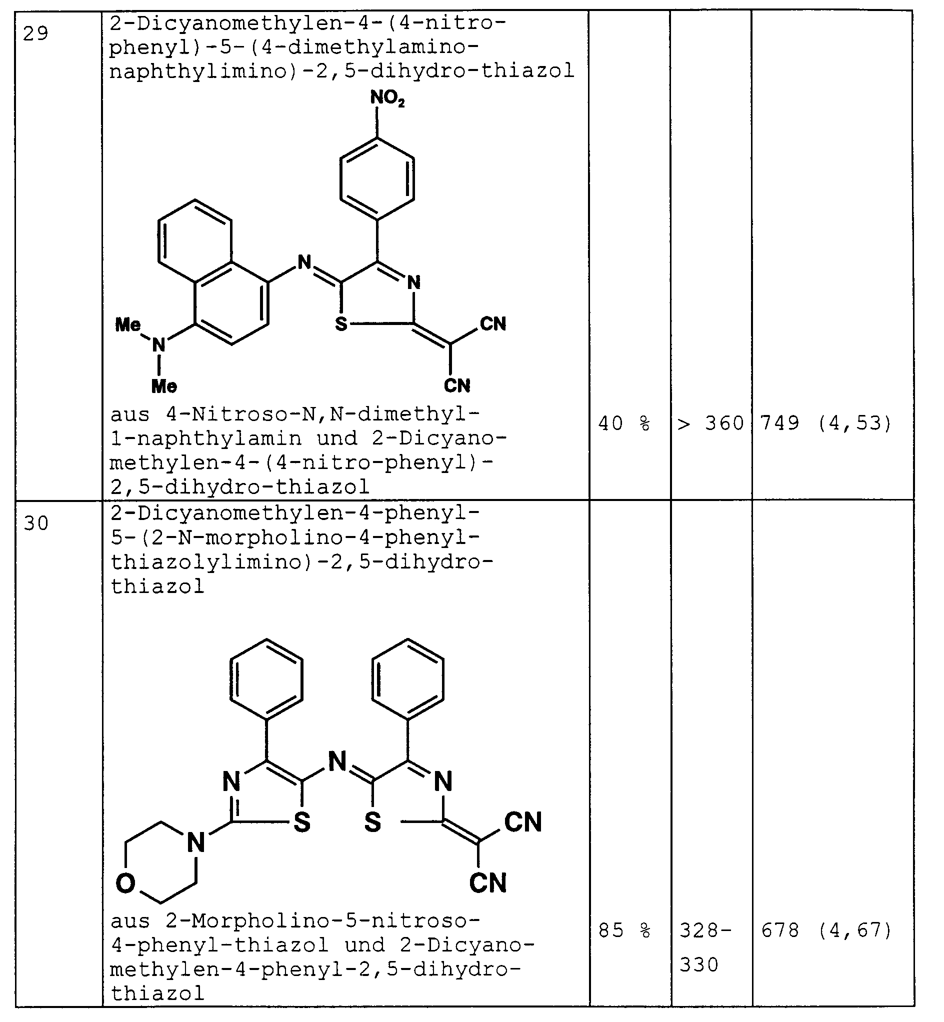

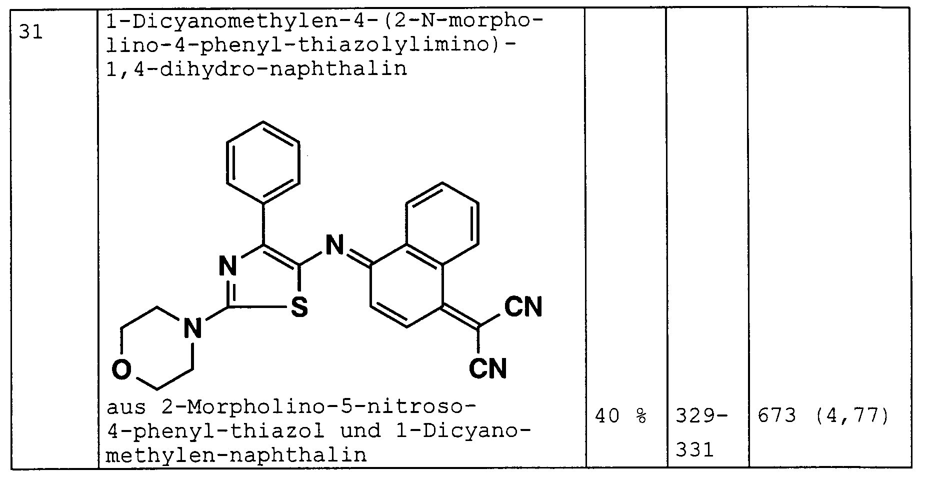

Die Azamethine gemäß der vorliegenden Erfindung lassen sich überraschend einfach und in hoher Reinheit durch das erfindungsgemäße Verfahren erzeugen. Auf Grund ihrer chromophoren Eigenschaften und ihrer guten Wärmestabilitat eignen sie sich zur Verwendung als Chromophorbausteine in NLO-aktiven Systemen.

## Patentansprüche

1. Verfahren zur Herstellung von Azamethinen der allgemeinen Formel 1 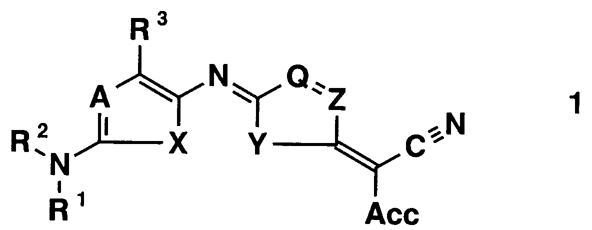 **dadurch gekennzeichnet,** daß man gemäß der folgenden Reaktionsgleichung 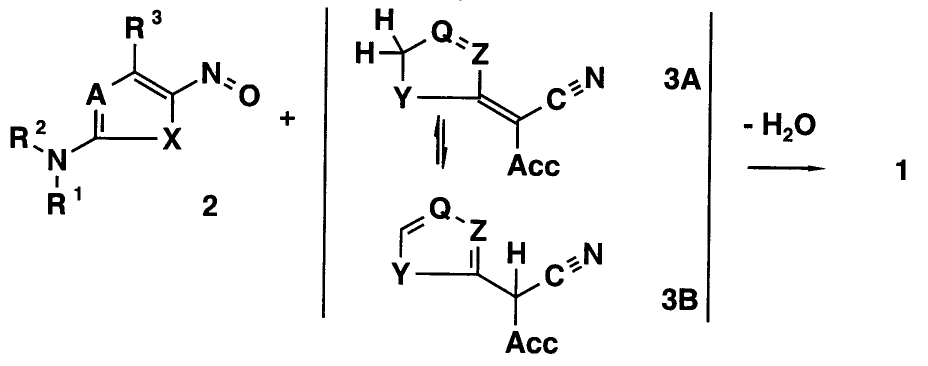 eine Verbindung der allgemeinen Formel 2 mit einer Verbindung der allgemeinen Formel 3A/3B in einem Lösungsmittel bei einer Temperatur zwischen 60 und 120°C kondensiert und das Kondensationsprodukt isoliert,
wobei in den Formeln 1 und 2
R¹ und R² - gleich oder verschieden voneinander - jeweils ein Wasserstoffatom, ein gerader oder verzweigter C₁- bis C₂₀-Alkylrest, ein Phenyl-, Naphthyl-, Thienyl-, Thiazolyl- oder Pyridylrest sind oder zusammen einen fünf- oder sechsgliedrigen carbocyclischen oder heterocyclischen Ring ausbilden können;
R³ ein Wasserstoffatom, eine Hydroxyl- oder O-Acylgruppe, ein gerader oder verzweigter C₁- bis C₂₀-Alkylrest oder ein gegebenenfalls in para-Position mit einer Halogen-, Hydroxyl- oder O-Acylgruppe substituierter Phenylrest ist;
X für S, Se, O oder NR⁴, wobei R⁴ ein Wasserstoffatom oder ein gerader oder verzweigter C₁- bis C₂₀-Alkylrest ist, oder für eine Ringdoppelbindung oder einen 1,2-anellierten Benzolring steht;
A für eine CH- oder CR⁵-Gruppe, wobei R⁵ ein gerader oder verzweigter C₁- bis C₂₀-Alkylrest, ein Phenyl- oder ein Naphthylrest ist, oder für N steht;
und in den Formeln 1 und 3A/3B
Y für S, Se, O oder NR⁶, wobei R⁶ ein Wasserstoffatom oder ein gerader oder verzweigter C₁- bis C₂₀-Alkylrest ist, oder für eine Ringdoppelbindung oder einen 1,2-anellierten Benzolring steht;
Q und Z - gleich oder verschieden voneinander - jeweils für eine CH- oder CR⁷-Gruppe, wobei R⁷ ein C₁- bis C₂₀-Alkylrest, ein gegebenenfalls substituierter Phenyl- oder ein Naphthylrest ist, oder für N stehen oder zusammen für eine Ringdoppelbindung oder einen 1,2-anellierten Benzolring stehen, mit der Maßgabe, daß Y nicht gleichzeitig ein 1,2-anellierter Benzolring ist, und
Acc eine Nitril-, C₁- bis C₂₀-Alkoxycarbonyl-, Formyl-, Acyl-, Arylsulfonyl- oder Nitrogruppe ist.

2. Verfahren nach Anspruch 1, **dadurch gekennzeichnet,** daß als Lösungsmittel für die Kondensation Anhydride niederer Carbonsäuren, dipolare aprotische Lösungsmittel oder Säurehalogenide in einem inerten Lösungsmittel eingesetzt werden.

3. Verfahren nach Anspruch 1 oder 2, **dadurch gekennzeichnet,** daß das Lösungsmittel für die Kondensation Acetanhydrid, Dimethylformamid, Dimethylacetamid, N-Methylpyrrolidon oder Phosphoroxychlorid oder Thionylchlorid in Dichlorethan oder Perchlorethylen ist.

4. Verfahren nach einem der Ansprüche 1 bis 3, **dadurch gekennzeichnet,** daß man das mineralsaure Salz 2·HAn einsetzt und zur Kondensation wenigstens eine äquivalente Menge einer Base zusetzt.

5. Verfahren nach Anspruch 4, **dadurch gekennzeichnet,** daß die Base Triethylamin oder Natriumacetat ist.

6. Azamethine der allgemeinen Formel 1, 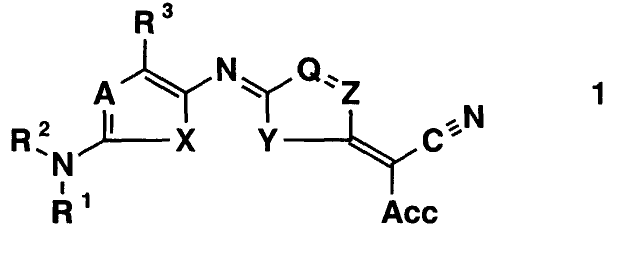 worin
R¹ und R² - gleich oder verschieden voneinander - jeweils ein Wasserstoffatom, ein gerader oder verzweigter C₁- bis C₂₀-Alkylrest, ein Phenyl-, Naphthyl-, Thienyl-, Thiazolyl- oder Pyridylrest sind oder zusammen einen fünf- oder sechsgliedrigen carbocyclischen oder heterocyclischen Ring ausbilden können;
R³ ein Wasserstoffatom, eine Hydroxyl- oder O-Acylgruppe, ein gerader oder verzweigter C₁- bis C₂₀-Alkylrest oder ein gegebenenfalls in para-Position mit einer Halogen-, Hydroxyl- oder O-Acylgruppe substituierter Phenylrest ist;
X für S, Se, O oder NR⁴, wobei R⁴ ein Wasserstoffatom oder ein gerader oder verzweigter C₁- bis C₂₀-Alkylrest ist, oder für einen 1,2-anellierten Benzolring steht;
A für eine CH- oder CR⁵-Gruppe, wobei R⁵ ein gerader oder verzweigter C₁- bis C₂₀-Alkylrest, ein Phenyl- oder ein Naphthylrest ist, oder für N steht;
Y für S, Se, O oder NR⁶, wobei R⁶ ein Wasserstoffatom oder ein gerader oder verzweigter C₁- bis C₂₀-Alkylrest ist, oder für einen 1,2-anellierten Benzolring steht;
Q und Z - gleich oder verschieden voneinander - jeweils für eine CH- oder CR⁷-Gruppe, wobei R⁷ ein C₁- bis C₂₀-Alkylrest, ein gegebenenfalls substituierter Phenyl- oder ein Naphthylrest ist, oder für N stehen oder zusammen für eine Ringdoppelbindung stehen, und
Acc eine Nitril-, C₁- bis C₂₀-Alkoxycarbonyl-, Formyl-, Acyl-, Phenylsulfonyl-, Naphthylsulfonyl- oder Nitrogruppe ist.

7. Azamethine nach Anspruch 6, **dadurch gekennzeichnet,** daß R¹ und R² gerade oder verzweigte C₁- bis C₂₀-Alkylreste sind, welche - gleich oder verschieden voneinander - durch 1 bis 5 Sauerstoffatome in Etherfunktion unterbrochen sind.

8. Azamethine nach Anspruch 6 oder 7, **dadurch gekennzeichnet,** daß ein aus R¹ und R² gebildeter carbocyclischer oder heterocyclischer Ring zusätzlich mit Hydroxy- und/oder C₁- bis C₂₀-Alkylresten substituiert ist.

9. Azamethine nach einem der Ansprüche 6 bis 8, **dadurch gekennzeichnet,** daß R¹ und R² zusammen einen Morpholin-, Piperidin-, Pyrrolidin- oder Piperazinring bilden.

10. Azamethine nach Anspruch 9, **dadurch gekennzeichnet,** daß R¹ und R² zusammen einen Morpholin-4-yl-, Piperazin-1-yl- oder 4-(C₁- bis C₄-Alkyl)-piperazin-1-yl-Rest bilden.

11. Azamethine nach Anspruch 10, **dadurch gekennzeichnet,** daß R¹ und R² zusammen einen 4-Methyl-piperazin-1-yl- oder 4-(2-Hydroxyethyl)-piperazin-1-yl-Rest bilden.

12. Azamethine nach einem der Ansprüche 6 bis 11, **dadurch gekennzeichnet,** daß R³ ein Wasserstoffatom oder ein substituierter Phenylrest ist.

13. Azamethine nach einem der Ansprüche 6 bis 12, **dadurch gekennzeichnet,** daß X für S, Se oder N steht.

14. Azamethine nach einem der Ansprüche 6 bis 13, **dadurch gekennzeichnet,** daß Y für S oder Se steht.

15. Azamethine nach einem der Ansprüche 6 bis 14, **dadurch gekennzeichnet,** daß Q und Z zusammen eine Ringdoppelbindung bilden oder Z für N und Q für eine CR⁷-Gruppe steht, wobei R⁷ ein gegebenenfalls substituierter Phenylrest ist.
